(19) **Europäisches Patentamt / European Patent Office / Office européen des brevets**

(11) **EP 4 528 541 A1**

(12) **EUROPEAN PATENT APPLICATION**

(43) Date of publication:
26.03.2025 Bulletin 2025/13

(21) Application number: 23198408.9

(22) Date of filing: 19.09.2023

(51) International Patent Classification (IPC):
$G06F\ 16/55^{(2019.01)}$     $G06N\ 3/045^{(2023.01)}$
$G06N\ 3/084^{(2023.01)}$     $G06N\ 3/09^{(2023.01)}$
$G06N\ 3/094^{(2023.01)}$     $G06V\ 10/82^{(2022.01)}$

(52) Cooperative Patent Classification (CPC):
G06N 3/045; G06F 16/55; G06N 3/09; G06N 3/094;
G06V 10/82; G06N 3/084; G06V 2201/03

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: FUJITSU LIMITED
Kawasaki-shi, Kanagawa 211-8588 (JP)

(72) Inventors:
• GEORGESCU, Serban
Slough, SL1 2BE (GB)
• CHAUDHURI, Abhra
Slough, SL1 2BE (GB)

(74) Representative: Haseltine Lake Kempner LLP
Cheapside House
138 Cheapside
London EC2V 6BJ (GB)

Remarks:
Amended claims in accordance with Rule 137(2) EPC.

(54) **A COMPUTER-IMPLEMENTED METHOD, DATA PROCESSING APPARATUS, AND COMPUTER PROGRAM FOR IMAGE ANALYSIS**

(57) A computer-implemented method of training a machine-learning model for image analysis, the method comprising receiving an input training dataset comprising real training data and augmented training data; iteratively training an encoder of the machine-learning model to obtain a trained encoder, the iterative training comprising: training a discriminator model by minimising a discriminator loss function using the input training dataset to obtain a trained discriminator, the trained discriminator being configured to discriminate whether input data is real data or augmented data; and training the encoder by maximising a discriminator error using the training dataset and the trained discriminator to obtain a trained encoder, the trained encoder being configured to invariantly encode real data and augmented data to a same representation space.

$T_i$: Transformer
$F_i$: Fusion
$R_i$: Reassemble

Figure 4

**Description**

**Field of the Invention**

The present invention relates to methods of image analysis in the field of computer vision. More particularly, the present invention relates to a method of training a machine-learning model for image analysis, a method of image analysis, and a related data processing apparatus, computer program, and computer-readable storage medium.

**Background of the Invention**

The computer vision technique of image segmentation refers to the process of partitioning an image into image segments, through classification of pixels in the image and the marking or masking the target region. Image segmentation is a widely applied technique in technical fields such as medical image analysis, object detection in unmanned driving, and geographic and geologic monitoring.

For instance, in the field of geographic monitoring, flood water monitoring is an active area of research. Flood monitoring through real-time video camera feeds and though analysis of image datasets typically requires a segmentation machine-learning model. The obtained segments or masks may be used to determine the extent of flood, which may then be used to inform emergency alerts and disaster management systems.

Floods are the most common natural disaster in the UK and have been identified as the most damaging form of natural disaster in Europe, having caused estimated damages worth thousands of millions of euros over recent years. Flood detection is viewed as an important safety measure and has received widespread interest from various research and technology groups worldwide. Existing approaches for detecting floods are generally based on sensors, satellite imagery, and video camera feeds.

However, sensors and satellite imagery come with certain drawbacks - sensors may be expensive and difficult to install. Satellite and airborne optical techniques are hampered by their daylight-only application and their inability to map flooding beneath clouds and vegetation. SAR imaging may alleviate some of the above limitations, but SAR satellite overpasses are infrequent (at most once or twice per day), so it is uncommon to capture the rising limb of the water.

As a result, river video cameras have emerged as a popular alternative. Advantageously, these provide continuous broadcast of live images, require low maintenance and installation costs, and enable easy communication of feeds through standard and/or mobile broadband.

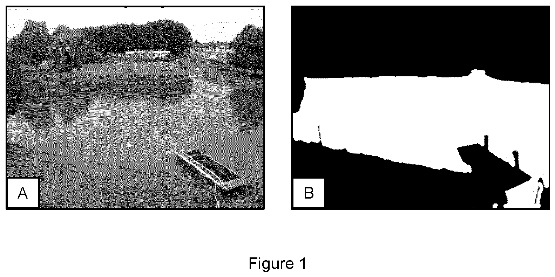

Leveraging river video camera feeds typically requires use of a water segmentation model. FIGURE 1 provides an example of application of a water segmentation model, adapted from the work of Vandaele, R. et al. (Hydrology and Earth System Sciences. 25. 4435-4453. 10.5194/hess-25-4435-2021). Panel A shows an image of a river (note that the original image includes colour data); panel B shows the segmentation results, masking the areas of concern that the water segmentation model classifies as water in white.

In the context of water segmentation, the current state-of-the-art for segmentation is believed to be achieved using AquaNet (the reader is referred to the work of Erfani, S. et al, ATLANTIS: A benchmark for semantic segmentation of waterbody images. Environmental Modelling & Software, 105333), which uses the PSPNet, a convolutional neural network (CNN)-based model as the network backbone.

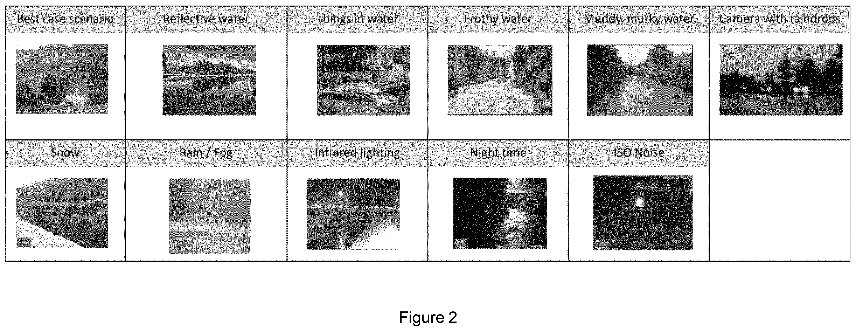

However, as water may appear in significantly different forms, the collection of sufficient real data to faithfully represent all forms is infeasible. FIGURE 2 provides an array of the variation in water images that a water segmentation model may encounter, including variations in image quality relative to a best case scenario (increased ISO noise, camera view obscured by raindrops), variations in image acquisition technique (infrared lighting), variations in lighting quality (reflective water, night time image acquisition), presence of precipitation (snow, rain, fog), and the nature of the water of interest itself (muddy or murky water, frothy water, water with objects submerged).

An ideal water segmentation model should thus generalise to all Out-of-Distribution (OoD) scenarios in which water may appear, despite only having access to a subset of real, In-Distribution images during inference. Simulations or augmentations that synthetically model OoD scenarios are able to help bridge this gap and address data scarcity concerns through the generation of artificial images in a controlled manner. Although structurally similar to real images, such artificial images exhibit a modality shift due to fundamental differences in the data-generation process from that of the real data.

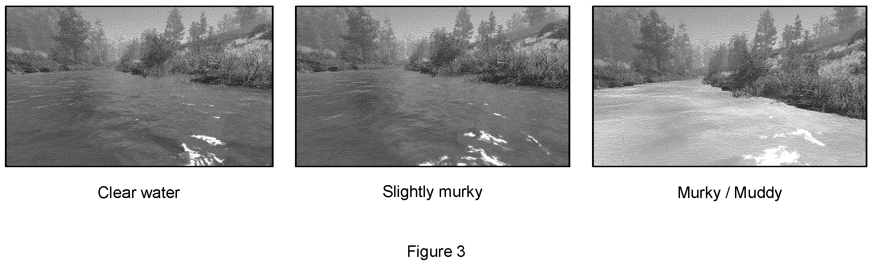

FIGURE 3, for example, demonstrates simulation of the variation in water colour attributes (note, again, that the original image includes colour data). The murkiness of the water is simulated via manipulation of colour in the image.

Existing approaches for ensuring segmentation models generalise (or are generalisable) to multiple modalities do so in the context of domain generalisation and adaptation. Known techniques have provided evidence that synthetic images may be used to bridge the datascarcity problem in computer vision tasks like object detection for grasping in robots. However, the inventors have come to the realisation that techniques that consider downstream OoD segmentation or classification tasks (rather than domain generalisation and adaption) have not been explored.

Of course, this problem of data scarcity for downstream OoD segmentation tasks is not limited to the field of flood management. Although existing approaches for combining real and simulated data for training large-scale image segmentation models (and, indeed, other image analysis models) are known, it is desirable to develop techniques to ensure efficient utilisation of first modality data (simulated or otherwise augmentative data) where the downstream task is known to be performed on distinct modality data.

**Summary of the Invention**

The invention is defined in the independent claims, to which reference should now be made. Further features are set out in the dependent claims.

According to an aspect of the invention, there is provided a computer-implemented method of training a machine-learning model for image analysis. The method includes a step of receiving an input training dataset, which includes real training data or, equivalently, training data corresponding to, acquired from or representative of a first modality. The input training dataset further includes augmented training data (or, equivalently, training data corresponding to, acquired from, or representative of a second, distinct, modality). The augmented training data may be thought of as augmenting or supplementing the information derivable from the real training data. The augmented training data may be manipulated real data, simulated or synthetic data, or otherwise supplementary. As an example in the context of image analysis in the form of water segmentation, real training data may be real, acquired, unaltered images of water in various environments, and augmented data may be synthetic images, either wholly artificially generated or synthesised by manipulating real images of water (e.g., through colour or texture manipulation).

The term "modality" in this context refers to the manner in which data are experienced or captured. The real modality thus refers to the manner in which real training data (and real data) are captured. Augmented data similarly may correspond to an augmented modality, distinct from the real modality. While not a necessity, augmented data may be also be "real" in the sense that the data are genuine or not artificial. The augmented modality is distinct from the real modality.

The method also includes iterative training of an encoder of the machine-learning model. That is, the (full) machine-learning model for image analysis includes an encoder component. Broadly, the encoder takes an input sequence (e.g., image data) and converts the sequence into a fixed-size representation or context vector (or embedding or projection). This representation ideally contains pertinent information derived from the input sequence and serves as a high-level summary of the input. By training the encoder until convergence (that is, optimising weights and biases and other parameters), a trained encoder may be obtained. The trained encoder may be referred to as a non-commutative invariant (NCI) encoder.

The iterative training of the encoder includes training of a discriminator model and training of the encoder itself. Training the discriminator model may be performed by minimising a first loss function value (a discriminator loss function). In effect, the discriminator model may be thought of a classifier. Computational means may pass the input dataset into the discriminator (in the form of representations or encodings acquired from the encoder following encoding of the input data), and the discriminator may undergo reinforcement learning and/or adversarial processing to classify both real training data and augmented data. The discriminator loss function penalises the discriminator for misclassification of real training data as augmented and augmented training data as real. The discriminator may, for example, update its weights and biases through backpropagation from the discriminator loss function through the discriminator model. The trained discriminator model is configured to discriminate whether new, unseen input data (and training data) is real data or augmented data.

Training the encoder may be performed by maximising a second loss function (a discriminator error, or an encoder loss function). The aim here is configure the encoder such that the discriminator is partially fooled, in the sense that real data is mapped to a representation space (encoding space or latent space) corresponding to real data and augmented data is also mapped to the representation space corresponding to real data. The invariance (in the encoder) achieved by this process is therefore non-commutative. Thus, the trained encoder is configured to invariantly encode real data and augmented data to a same, real representation space (corresponding to the real modality).

The trained encoder therefore maps augmented data to the real representation space, and does not map real data to an augmented representation space. In this sense, the trained encoder encodes or produces representations in a non-commutative manner. The trained encoder fixes the direction of invariance towards the real data modality. While known techniques learn invariances by eliminating modality-specific information from all modalities (both real and augmented), embodiments make the learning invariant to the augmented modality, preserving all real modality-specific information in the process. Learning the invariance in a non-commutative manner therefore preserves the real modality information. Methods according to embodiments therefore offer an approach to efficiently leverage or use augmented data to train large-scale models, such as vision transformers for achieving OoD generalisation, in scenarios where limited amounts of real data are available.

The performance of machine-learning models, such as deep neural networks, is known to scale with training dataset sizes, a fact that is especially true for encoder-based architectures. The present disclosure enables scaling of training dataset sizes in a manner that aids the generalisation abilities of image analysis machine learning models for such varied use cases as water segmentation and medical image classification.

As the modality discriminator trains, the discriminator improves in being able to distinguish between real and augmented modalities. Training the encoder to fool the discriminator forces the former to produce representations that do not contain synthetic modality-specific information.

Related work includes Gradient Reversal Layer (GRL) for domain adaptation (the reader is directed to the work of Ganin, Y. et al. Domain-adversarial training of neural networks. J. Mach. Learn. Res. 17, 1 (January 2016), 2096-2030). GRL forces a backbone encoder to be robust to modality shifts by enforcing a commutative form of invariance. In other words, GRL techniques try to fool a discriminator by inverting the modality labels, making the model invariant to both real and synthetic modality information. However, when modality shifted data is used as train-time augmentations, it is not necessary to enforce bidirectional (commutative) invariance (as imposed in GRL techniques). That is, this commutativity is unnecessary when data from another modality is used as an augmentation to a training data set (and not encountered during inference). Instead, by making the encoder maximise the discriminator error by only mapping augmented data to the real representation space, and not the other way around, models trained in accordance with the present disclosure are able to use modality shifts as a regularizer to make encoder-based models leverage multi-modal data-sources to aid OoD generalisation. This leads to a significantly more parameter efficient model. Combining real and augmented data in a modality-invariant manner helps the downstream segmentation and/or classification accuracy. In an example of encoder-based (or transformer encoder-based) segmentation models, sampling from the proposed Non-Commutatively Invariant (NCI) representation space enables improvement in both in-distribution and OoD segmentation performance. With modality invariance, encoders are able to better utilise the increase in the number of synthetic samples.

Optionally, the machine-learning model may comprise further downstream layers. That is, the machine-learning model may comprise layers in addition to the encoder. When trained, the further downstream layers along with the trained encoder may be configured (by training) to output analysis results of input data. The input data corresponds to the same (or a similar) real modality as that of the real training data.

Optionally, the method of training a machine-learning model for image analysis may further comprise iterative training of any further downstream layers. For instance, a multi-objective optimisation procedure (or a Pareto optimisation) may be used to simultaneously optimise the encoder and any further layers.

Optionally, the machine-learning model may comprise transformer encoders, reassembly modules, and fusion modules. In turn, when trained, the trained machine-learning model may comprise trained transformer encoders, trained reassembly modules, and trained fusion modules. This configuration is well suited to processing of representations at a constant and relatively high resolution and ensures a global receptive field at every stage. These properties allow the machine-learning model to provide finer-grained and more globally coherent predictions when compared to fully-convolutional networks.

Optionally, the method may comprise use of the trained machine learning model. That is, the method may include receiving an input image (or multiple images). This input image may be input from a repository of images, such as from a database. Additionally or alternatively the input image may be input in real-time, for instance, from a live video feed. The method may then process the input image using the trained machine-learning model to obtain analysis results (e.g., image segments or image classifications) of the input image. The input data corresponds to the same (or a similar) real modality as that of the real training data. In this way, inference may be performed on the input data in a manner that considers OoD generalisation, using information derived from the augmented training data.

According to a further aspect of the invention, there is provided a computer-implemented method of image analysis. The method receives an input image and - using a machine-learning model trained in accordance with the techniques discloses above and further herein - obtains analysis results.

Broadly, techniques herein provide an efficient way of utilising data-augmentations for any kind of task that requires invariance to the augmented modality.

For instance, optionally, the method of image analysis and/or the method of training a machine-learning model for image analysis are/is for water image segmentation. The input training dataset in this event therefore comprises real training water images and augmented or simulated training water images. Since water may appear very differently under different conditions, collecting enough real images that faithfully represent all such conditions is infeasible. Techniques herein successfully identify synthetic data to be a viable alternative to real data for modelling distribution shifts in the appearance of water.

Optionally, when the methods are for water image segmentation, the input water image may be acquired in real-time (such as from a live video stream). The real-time image(s) may be processed using the trained machine-learning model to obtain water segmentation results.

Optionally, water segmentation results may be used to determine whether it is necessary to output an alarm. For instance, the water segmentation results (e.g., a water image segment) (or derivate data thereof) may be compared to a threshold. For instance, a segment occupying 15% of an image frame may be compared to a predefined threshold of 10%, which initiates the output of the alarm. The alarm may be an audible signal, or may be a data signal for transmission to the relevant authorities or personnel.

Combining real and augmented water images to train a machine-learning water segmentation model provides significant performance gains over direct mixing of the two modalities. Techniques herein provide a state-of-the-art model for water segmentation, which may be used to inform emergency alert and disaster management systems dealing with flood, one of the most common sources of natural disasters across.

Optionally, the method of image analysis and/or the method of training a machine-learning model for image analysis are/is for medical image analysis, such as segmentation or classification. The input training dataset in this event may therefore comprises training medical images and augmented medical data, such as genomic data corresponding to the same subject as the subjects of the medical images. Genetic or genomic information along with easy-to-obtain non-genetic data (like medical images) may be available during training, but only the latter may be available during inference.

In such multi-modal genomics applications, existing approaches that use genetic information as train-time augmentation data learn commutative forms of invariances via contrastive learning. Use-cases arising in multi-modal genomics applications. For multi-modal genomics applications where both genetic information and medical images may be used only during training, models may learn a non-commutative invariance from the genetic modality to the image modality, thus leveraging the complementary semantics provided by the genetic data, while discarding its modality-specific information. The non-commutativity of the invariance allows improved inference efficiency during real-world deployment, which is typically performed on imaging data only, without access to any genetic information.

Optionally, when the methods are for medical image segmentation or classification, the input medical image may be acquired in real-time (such as from a live video stream, or live medical scan). In this way, image segmentation may be efficiently performed in-situ, such that adaptive radiotherapy or other treatment techniques may be implemented immediately. Similarly, in this way, image classification may be performed immediately.

Embodiments of another aspect include a data processing apparatus comprising a memory storing computer-readable instructions and a processor. The processor (or controller circuitry) is configured to execute the instructions to carry out the computer-implemented method of training a machine-learning model for image analysis and/or to carry out the computer-implemented method of image analysis.

Embodiments of another aspect include a computer program comprising instructions, which, when executed by computer, causes the compute to execute the computer-implemented method of training a machine-learning model for image analysis and/or to carry out the computer-implemented method of image analysis.

Embodiments of another aspect include a non-transitory computer-readable storage medium comprising instructions, which, when executed by a computer, cause the compute to execute the computer-implemented method of training a machine-learning model for image analysis and/or to carry out the computer-implemented method of image analysis.

The invention may be implemented in digital electronic circuitry, or in computer hardware, firmware, software, or in combinations thereof. The invention may be implemented as a computer program or a computer program product, i.e., a computer program tangibly embodied in a non-transitory information carrier, e.g., in a machine-readable storage device or in a propagated signal, for execution by, or to control the operation of, one or more hardware modules.

A computer program may be in the form of a stand-alone program, a computer program portion, or more than one computer program, and may be written in any form of programming language, including compiled or interpreted languages, and it may be deployed in any form, including as a stand-alone program or as a module, component, subroutine, or other unit suitable for use in a data processing environment. A computer program may be deployed to be executed on one module or on multiple modules at one site or distributed across multiple sites and interconnected by a communication network.

Method steps of the invention may be performed by one or more programmable processors executing a computer program to perform functions of the invention by operating on input data and generating output. Apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The invention is described in terms of particular embodiments. Other embodiments are within the scope of the following claims. For example, the steps of the invention may be performed in a different order and still achieve desirable results.

Elements of the invention have been described using the terms "processor", "input device" The skilled person will appreciate that such functional terms and their equivalents may refer to parts of the system that are spatially separate but combine to serve the function defined. Equally, the same physical parts of the system may provide two or more of the functions defined. For example, separately defined means may be implemented using the same memory and/or processor as appropriate.

**Brief Description of the Drawings**

Reference is made, by way of example only, to the accompanying drawings in which:

Figure 1 is an example of image analysis in the form of water segmentation;
Figure 2 is a collection of example OoD scenarios in water segmentation;
Figure 3 is an example of image augmentation, demonstrating control of a water colour attribute through simulation or synthesis;
Figure 4 is schematic representation of a DPT-based machine-learning architecture for water segmentation;
Figure 5 is a representation of modality shifts in representation space caused via conventional encoders;
Figure 6 is a summary of NCI techniques implemented according to embodiments;
Figure 7 is a flowchart indicating a general method of training a machine-learning model for image analysis according to embodiments;
Figure 8 is an example training process for a water segmentation model with NCI according to embodiments;
Figure 9 is an example training process for a medical image classification model with NCI according to embodiments;
Figure 10 is a flowchart indicating a general method of image analysis according to embodiments;
Figure 11 is an example inference process for a water segmentation model with NCI according to embodiments;
Figure 12 is an example inference process for a medical image classification model with NCI according to embodiments;
Figure 13 is a further example training process for a water segmentation model with NCI according to embodiments;
Figure 14 is a further example training process for a medical image classification with NCI according to embodiments;
Figure 15 is a collection of water images and corresponding water segments, determined according to a trained model, trained according to embodiments; and
Figure 16 is a block diagram of computational means for implementation of methods according to embodiments.

**Detailed Description**

The inventors show that learning modality invariant representations is a good way to leverage data augmentations that come from a different modality, such as those from simulations.

In the context of water segmentation, as mentioned above, the current state-of-the-art for segmentation is achieved using the AquaNet model. However, as will become evident, the inventors have demonstrated that CPNet (the reader is directed to the work of Yu, C. et al. (2020) Context Prior for Scene Segmentation. Proceedings of the IEEE Conference on Computer Vision and Pattern Recognition (CVPR)) performs better for water segmentation with OoD scenarios. Further, the inventors also demonstrate that use of DPT - one of the most commonly used transformer models for segmentation (the reader is directed to the work of Ranftl, R. et al. (2021) Vision Transformers for Dense Prediction. ICCV, 2021) - surpasses the performance of the aforementioned models for OoD generalisation for water segmentation by over 12%. The following discussion therefore considers DPT as the baseline segmentation model, which is also used as an example to illustrate currently existing inefficiencies.

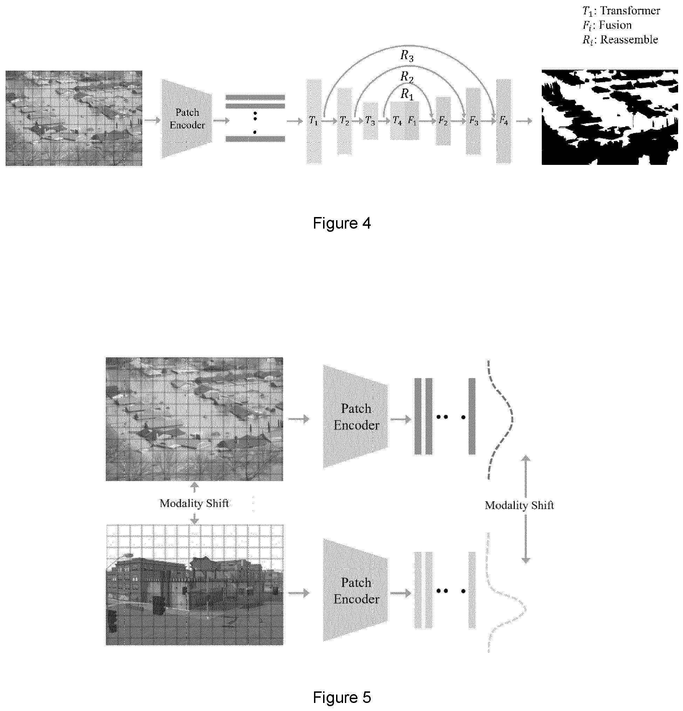

FIGURE 4 illustrates the architecture of the DPT model as applied in the context of water segmentation. When the model is trained, the input image is transformed into tokens after passing the input image through a patch encoder. This transformation is performed either by extracting non-overlapping patches followed by a linear projection of their flattened representation (or embeddings or projections) or by applying a ResNet-50 feature extractor. Patches in this context are represented by the grid-like overlay on the input image. The image embedding may be augmented with a positional embedding and a patch-independent readout token may be added. The tokens are passed through multiple transformer stages ($T_1$, $T_2$, $T_3$, $T_4$). Tokens are then reassembled from different stages into an image-like representation at multiple resolutions, through reassembly operations ($R_1$, $R_2$, $R_3$). The reassembly operations may assemble tokens into feature maps with a fraction of the spatial resolution the input image. Fusion modules ($F_1$, $F_2$, $F_3$, $F_4$) progressively fuse and upsample the representations to generate a fine-grained prediction. Fusion modules combine features using residual convolutional units and upsample the feature maps. The results fine-grained prediction, as shown, is a segmentation mask corresponding to the input image.

However, as illustrated in FIGURE 5, the DPT model does not have provide any built-in mechanism to combine real and synthetic training data. Hence, the representations from the patch encoder are sensitive to the modality gaps between distinct sets of training data, which may harm inference and test time performance. That is, an image of a first modality (top, such as real data), when passed through the patch encoder (and potentially other layers of the DPT model), will result in a representation in one position in latent space or representation space. An image of a second modality (bottom, such as simulated data), when passed through the same patch encoder, will result in a representation in a distinct position in latent space.

In the absence of paired training data, the currently known best approach for combining from multiple

modalities is by learning modality-invariant representations using Gradient Reversal Layer (GRL). GRL forces the backbone encoder to be robust to modality shifts by enforcing a commutative form of invariance. In other words, GRL tries to fool a discriminator by inverting the modality labels, making the model invariant to, for example, both real and synthetic modality information.

As evidenced herein, this commutativity is unnecessary when data from the one modality (e.g., simulated data) is used only as an augmentation, and not encountered during inference. In the present case, techniques perform a non-commutative modality invariance, where only simulated data is mapped to the space of real data, and not the other way around. This leads to a significantly more parameter efficient model.

To summarise the above, the existing approaches for water segmentation suffer from the effects of distribution shifts in real-world water images. Although training models with simulated data that replicate distribution shifts may be a potential solution, state-of-the-art models for water segmentation do not have any built-in mechanism for consuming simulated data, and may exhibit inefficiencies induced by the modality gap between real and simulated data.

As mentioned above, techniques herein are not limited to the field of flood management. When applying computer vision in healthcare, such as when trying to automatically analyse eye fundus or radiography images, it is possible to create a training dataset that provides genetic information alongside the medical images. However, since sequencing a patient's DNA is a time-consuming and expensive process, it is thus not practical to expect the availability of genetic information during real-time diagnosis (inference). It is more reasonable to only train a model on genetic and medical images, where the genetic data acts as a source of augmentation (much like the synthetic or simulated images in the flood detection scenario). The medical image segmentation or classification model may then be deployed to perform inference, potentially in real-time, using easy-to-obtain medical images, while still leveraging the knowledge the model has learned by looking at the genetic information at train time. Hence, commutative invariance leads to a parameter inefficient model with multi-modal genomics use cases.

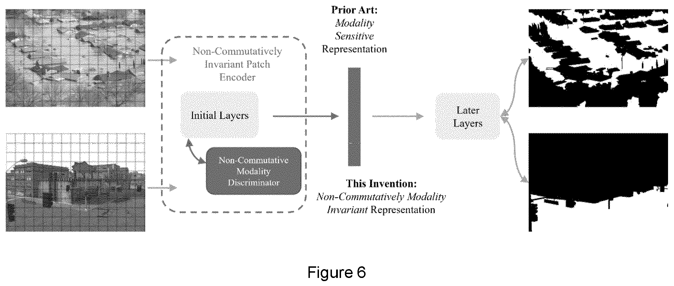

FIGURE 6 is a schematic diagram of the use of non-commutative invariance (NCI) for water segmentation, which efficiently leverages synthetic augmentations in training data. When trained with NCI representations (or embeddings or projections), otherwise generic segmentation models may better utilise train set augmentations (e.g., augmented or supplemented with synthetic samples) over known existing methods, such as combining multiple modalities. That is, training with synthetic data is an effective approach for achieving OoD generalisation in water segmentation.

As illustrated, the NCI encoder (that is, an encoder of an image segmentation machine-learning model, trained to enforce non-commutative invariance) processes images from distinct modalities. Known techniques result in modality sensitive representations; techniques that employ the NCI encoder result in non-commutatively modality invariant representation. Representations may then be processed by conventional later layers of networks to produce water segmentation masks.

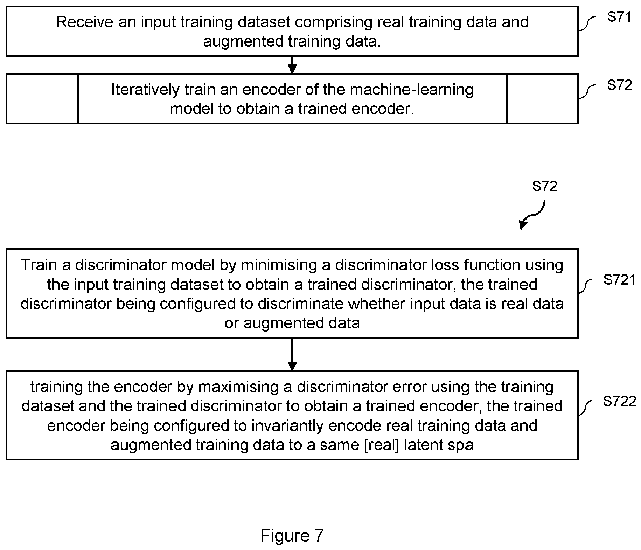

FIGURE 7 presents a general method for training a machine-learning model for image segmentation, which includes training of an encoder configured to invariantly encode data from a first modality and data from a second modality to a same representation space corresponding to the first modality.

At step 71, a computer receives an input training dataset, which includes real training data (that is, data for a first modality) and augmented training data (that is, data for a second modality, which may be referred to in varying contexts as other data, manipulated data, simulated data, supplementary data, genomic data, etc.).

At step 72, the computer performs training of the encoder in an iterative manner, until convergence of the encoder. In turn, this process results in a trained non-commutative invariant encoder.

The training process 72 involves, at step 721, training of a discriminator model. Training of the discriminator model includes minimisation of a discriminator loss function, and uses embeddings acquired from the encoder (following initialisation, and of course prior to final convergence of the encoder). The training process uses the input training dataset. When trained, the discriminator is configured to discriminate whether input data is real data (data of a first modality) or augmented data (data of a second modality).

The training process also involves, at step 722, training of the encoder through maximisation of a discriminator error. The training processes used the input training dataset and the trained discriminator (potentially not the final version of the trained discriminator; rather, the iterative process means that the weights of the discriminator may be updated in an iterative manner following update of weights of the encoder). When trained, the encoder is configured to invariantly encode real data and augmented data to a same, real representation space.

As discussed above, the outputs of the early layers of a machine-learning network that does not enforce modality invariance (patch encodings in the specific case of DPT, as discussed herein) are sensitive to modality shifts. Ideally, the early layers (patch representations for transformers) should covary with changing semantics and not with changing modalities. For example, in the context of water segmentation, early layers of a water segmentation model should covary with the absence/presence of flood water, and not with changing modes of image acquisition, changing light conditions, changing precipitation levels, etc. Making the early layer representations invariant to modality-specific information helps integrate a diverse set of multi-modal data-sources for training machine learning models, such as deep neural networks, for segmentation, for instance with large quantities of easily accessible simulated data. This is especially useful for transformer-based models, as their performance scales directly with the amount of training data. Modality invariance allows transformers to sample from a representation space that is "modality-agnostic", and only encodes the variations in semantics.

The techniques herein make early layer representations of the segmentation model invariant to the image modality (real/synthetic) from which the input derives. However, during evaluation or inference, the model will only encounter images of the real modality as synthetic data are only used as train-time augmentations. Thus, different from existing approaches of enforcing invariance that achieve their objective by removing modality specific information from all modalities, techniques herein map only synthetic images to the real representation space, without disturbing the representations of the real images. This leads to a significantly more parameter efficient model as the learning objective is simplified, and the model may leverage modality specific information (such as texture) corresponding to the real modality during inference.

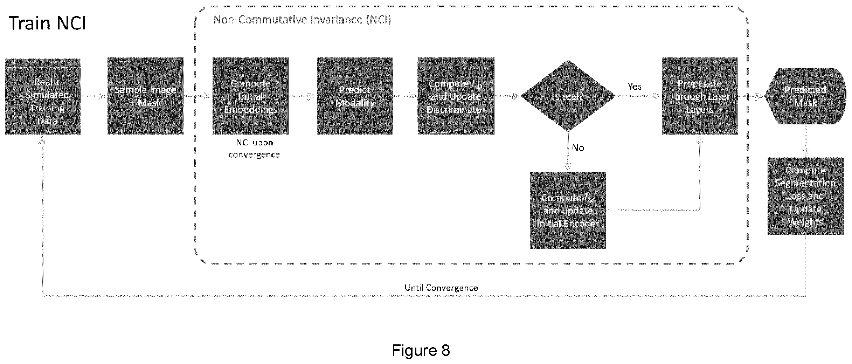

FIGURE 8 is a schematic diagram showing a training process for a segmentation model, trained with NCI. The proposed invariance is enforced on the embeddings obtained after propagating the input training image through the first few layers (initial encoder) of a neural network for segmentation. Input training images in this example are real training images and simulated training images. Training images are supplied with ground truth segmentation masks, and supplied with an indicator as to the modality (real or simulated).

The first step in enforcing non-commutative modality invariance is performed through introduction of a discriminator network, which may be adversarially trained to distinguish between real and the simulated or synthetic modalities, with fixed patch representations. That is, the encoder computing the patch representations for processing with the discriminator is fixed - encoder weights are not updated via backpropagation from the discriminator loss. The discriminator loss for each sample is calculated using the discriminator loss function, as follows:

$$L_D = y \log\Big(D\big(E(x)\big)\Big) + (1-y)\log\Big(1 - D\big(E(x)\big)\Big), \qquad \text{(Eqn. 1)}$$

where $x$ is the input image, $D$ is the discriminator which predicts the probability that the image is synthetic, E is the patch encoder, and y is the ground-truth modality label (0 for real and 1 for synthetic).

Once updated, the initial embedding layers are then trained to partially fool the modality discriminator (by removing modality specific information). This involves making the discriminator map synthetic images to representations that the discriminator perceives as being real. However, the real images are also mapped to the representation space where they are still considered real. The discriminator is partially fooled, in that the discriminator perceives real data as being real and perceives synthetic data as being real. The invariance achieved by this algorithm is thus non-commutative. The patch encoding loss is calculated using the discriminator error as follows:

$$L_E = y \log\Big(1 - D\big(E(x)\big)\Big) \qquad \text{(Eqn. 2)}$$

Of course, the skilled reader will appreciate that other forms of loss function are feasible, so long as the loss functions serve to minimise or maximise a first loss function value, which discriminates between modalities, and maximise or minimise a second loss function value, which encodes all modalities to a same representation space.

For the NCI learning, the discriminator weights, D, and the patch encoder weights, E, may be updated via a gradient descent method using the following assignments:

$$D \leftarrow D - \eta \nabla_D L_D \qquad \text{(Eqn. 3a)}$$

$$E \leftarrow E - \eta \nabla_E L_E \qquad \text{(Eqn. 3b)}$$

where $\eta$ is the learning rate, $\nabla_D$ is the gradient in the discriminator loss function, and $\nabla_E$ is the gradient in the discriminator error. Of course, distinct learning rates may be used for the distinct weight updates.

When trained, later layers of the segmentation model may then be trained using conventional techniques, albeit using NCI representations obtained through the trained NCI encoder.

As with water segmentation, NCI techniques may be applied to such use cases as multi-modal genomic image segmentation. The initial encoder may be optimised to minimize $L_E$ for datapoints from the genetic modality. This maps samples with genetic information only to the non-genetic (inference-time) modality in the representation or representation space.

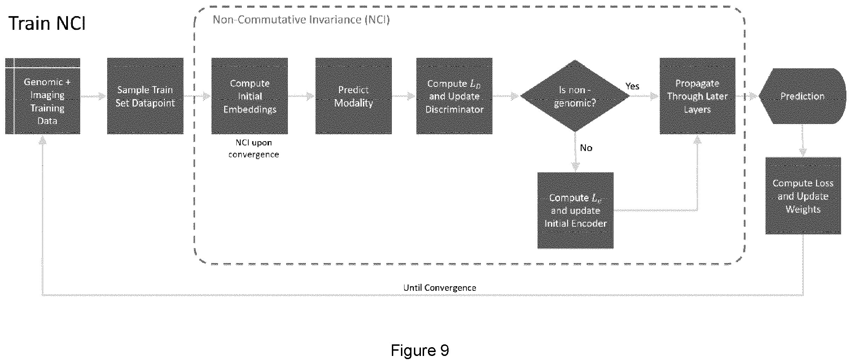

FIGURE 9 illustrates a training process for a segmentation or a classification model with a genomics use case, trained with NCI. The reader will appreciate that the logic is very similar to the training process described above, which involves real and simulated training data. Input training data in this example, however, are real medical images (such as X-rays, MRI scans, etc.) and corresponding genomic data (that is, genomic data of the same subject as the subject of the medical image). Training data are supplied with ground truth segmentation masks or classifications, and may be supplied with an indicator as to the modality (genomic or medical image). The discriminator network in this context may be adversarially trained to distinguish between the imaging modality and the genetic modality. In the case of a segmentation model, the trained model will output segments or masks for input medical images, where the segmentation is performed with additional context provided by the genomics training data. In the case of a classification model, the trained model will output classification of the input medical image, where the classification is performed with additional context provided by the genomics training data.

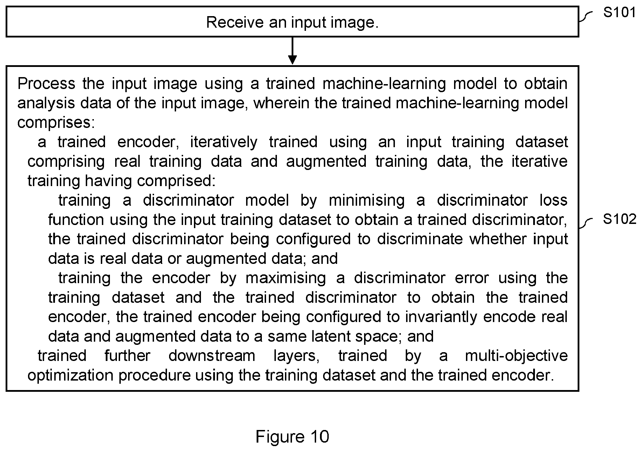

FIGURE 10 presents a general method for use of the trained machine-learning model for image analysis. The trained machine-learning model includes a trained encoder, configured to invariantly encode data from a first modality and data from a second modality to a same representation space corresponding to the first modality.

At step 101, a computer receives an input image. At step 102, the computer processes the input image using the trained machine-learning model to obtain analysis data of the input image.

The trained encoder of the trained machine-learning model is one that has been iteratively trained using an input training dataset comprising real training data and augmented training data. The iterative training will have included training a discriminator model by minimising a discriminator loss function using the input training dataset to obtain a trained discriminator, the trained discriminator being configured to discriminate whether input data is real data or augmented data. The iterative training will also have included training the encoder by maximising a discriminator error using the training dataset and the trained discriminator to obtain the trained encoder, the trained encoder being configured to invariantly encode real data and augmented data to a same representation space.

The trained machine-learning model also includes trained further downstream layers. These will have been trained using, for instance, a multi-objective optimisation procedure using the training dataset and using the trained encoder. As above, the trained further downstream layers will have been trained using conventional techniques, albeit using NCI representations obtained through the trained NCI encoder.

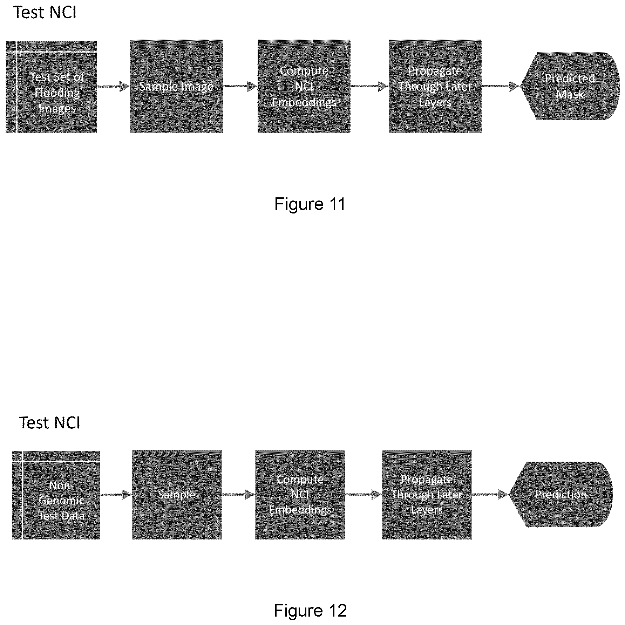

FIGURE 11 is a schematic diagram showing an inference (or a testing) process using the segmentation model trained with NCI, in the context of water segmentation. Inference is performed in a similar way as for conventional techniques, only with replacement of initial encoding layers with the NCI layers. The test set includes only real, non-simulated images of water. The trained segmentation model outputs prediction of masks or segments, indicative of the predicted presence of water in the test set images.

FIGURE 12 is a schematic diagram showing an inference (or a testing) process using the image analysis model trained with NCI, in the context of medical image classification. The test set includes only medical imaging data. However, the trained NCI encoder, as above, will have been trained with both medical imaging data and genomics data. In this way, the encoder learns a non-commutative invariance from the genetic modality to the image modality. The trained classification model outputs predictions of the presence of a genetic condition, as inferred from the test set images.

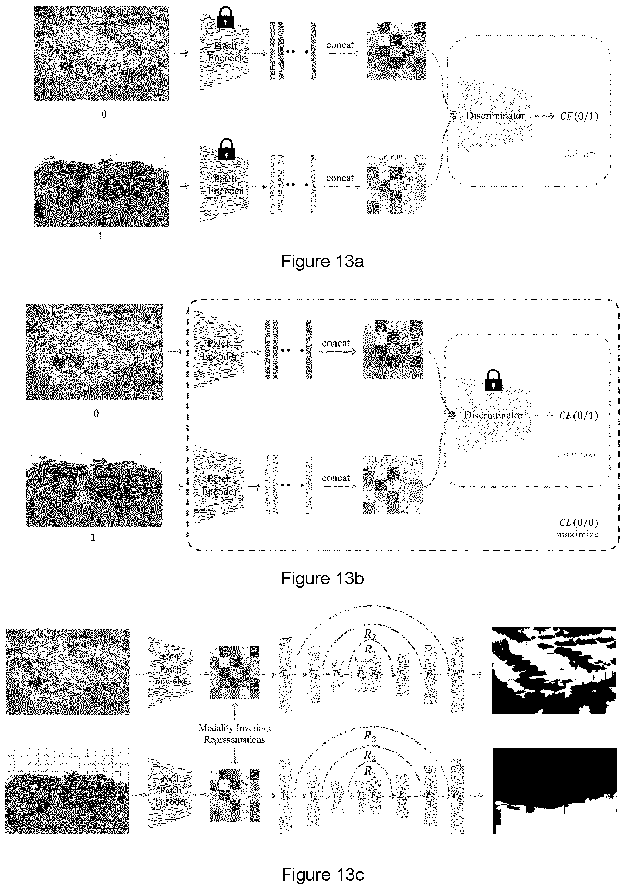

FIGURE 13 provides a worked example of NCI by training the DPT transformer model in a non-commutatively invariant manner for water segmentation. Training data in this context are, again, real image data (labelled "0") and simulated image data (labelled "1").

FIGURE 13a illustrates a first step in a training process. A computer trains a discriminator CNN on the patch representations from DPT. The patch representations may pass through further layers, the results of which may be concatenated before input into the discriminator. This training may be performed in an adversarial manner, such that the discriminator is able to classify between the real and the simulated modalities. This training involves minimisation of a discriminator loss function value. More specifically, this training involves minimisation of the cross-entropy loss during the adjustment of discriminator weights.

FIGURE 13b illustrates a second step in the training process. The computer trains the DPT patch encoder to maximise a discriminator error by mapping all representations to the "real" modality. The patch encoder thus becomes invariant to the modality information, but in a non-commutative manner. That is, simulated images are mapped to the real modality in the representation space, while the modality information in the real images is preserved. Training involves maximisation of the discriminator error by maximisation of the cross-entropy loss during the adjustment of encoder weights.

FIGURE 13c illustrates a third step in the training process. The non-commutatively invariant (NCI) patch representations are then used to train the downstream transformer layers of DPT. The discriminator has no role in this aspect of the training process. In effect, the trained patch encoder is "frozen" (i.e., the weights and biases thereof are frozen) and used in the full network training process. As shown, the effect of the trained NCI patch encoder is to encode images from either modality into a shared representation space, in a modality invariant manner.

Once trained, inference may be performed as usual, with the NCI patch encoder being used in place of the vanilla patch encoder.

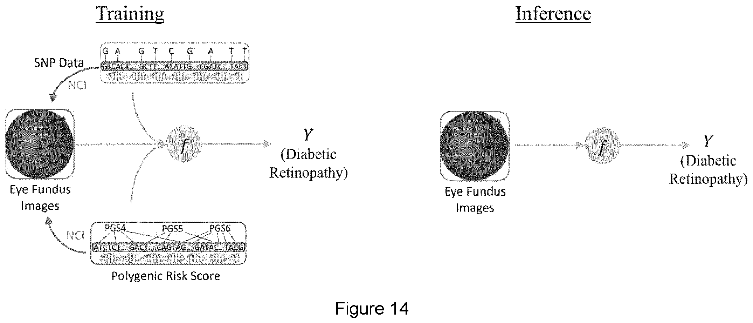

FIGURE 14 provides a worked example of NCI, used for medical image classification. Training data in this context are medical images (specifically, eye fundus images, obtained using OCT or similar) and genomics data.

For the prediction of diabetic retinopathy (a complication of diabetes, resulting in damage in the back of the eye), a multitude of genetic and non-genetic factors are known to be determinants. Various genetic data sources and acquisition modalities, such as single nucleotide polymorphisms (SNP) and Polygenic Risk Score (PRS), along with non-genetic fundus images are used for training models for predicting or classifying diabetic retinopathy. In practice, however, genetic data sources are not typically available for inference when such prediction or classification models are deployed in the real world. Hence, a non-commutative invariance (NCI) may be learned by mapping the SNP and/or PRS modalities to the imaging modality. This enables the eventual prediction or classification model to mine semantically relevant knowledge from both the genetic and imaging modalities, while discarding geneticsspecific information, which will not be encountered at test or inference time. During inference, only eye fundus images are provided to the model for the prediction of diabetic retinopathy. As shown, the input training dataset in this example comprises eye fundus images, labelled to indicate if the subject is known to suffer from diabetic retinopathy. The training dataset further comprises genomics data for the same subjects, in the form of SNP data and PRS data. Training of the encoder (here, denoted $f$) may be performed in a comparable manner to the training methods described above. When trained, the trained encoder may be implemented in a classification or image recognition model, such as an Inception-v3 deep CNN. This model - itself which may be trained in accordance with conventional techniques, albeit incorporating the trained encoder - may then be configured to accept eye fundus images from new, unseen subjects and output analysis results in the form of diabetic retinopathy classification or probability (y).

TABLE 1 below provides a summary of the quantitative performance of NCI when employed in a DPT model, for water segmentation. Performance is quantised using the Intersection over Union (IoU) metric - a metric that quantifies the degree of overlap between a predicted water segment (or segments) and ground truth segments.

| Training Dataset | Model | Dataset 1 (In-Dist) | Dataset 2 (OOD) |
|---|---|---|---|
| Real Only | AquaNet | 63.60 | 44.80 |
| Real Only | CPNet | 88.10 | 60.00 |
| Real Only | DPT | 87.95 | 72.57 |
| Real Only | Finetuned-DPT | 88.15 | 75.08 |
| Real + Synthetic Mixed (small) | Finetuned-DPT | 88.57 | 75.93 |
| Real + Synthetic Mixed (large) | Finetuned-DPT | 88.63 | 76.05 |
| Real + Synthetic Mixed | NCI-DPT | 88.25 | 76.69 |
| Real (Balanced) + Synthetic Mixed (small) | NCI-DPT | 88.97 | 76.89 |
| Real (Balanced) + Synthetic Mixed (large) | NCI-DPT | 89.02 | 77.49 |

All hyperparameters and experimental settings are also based on the Domain Adversarial Neural Networks Ganin & Lempitsky (2015) implementation of DomainBed (Gulrajani & Lopez-Paz, 2021), as that provides a fair ground for comparison with existing art. Namely, the following hyperparameters were found to be suitable for models trained in accordance with techniques (denoted NCI-DPT):

- Optimiser: Adam
- Learning rate: 0.001
- Scheduler: Exponential (with gamma set to 0.99)
- Weight decay: 0.0001
- Architecture specific choices like activation function, number of hidden units, layers, dropout rate, etc: a standard DPT with a ResNet50 as the patch encoder was used.
- Pretraining: the DPT with ResNet50 patch encoder, pretrained on ADE20K. The inventors finetuned the pretrained model, provided by the authors of DPT.
- Train-test split: the total number of real samples was around 4,000; the number of augmented simulated samples was varied from around 4,000 (small) to 12,000 (large). The test sets were fixed. The in-distribution test set had around 300 samples, and the out-of-distribution test set had around 600 samples.

As shown, of all the prior art models (AquaNet, CPNet, DPT, Finetuned-DPT), DPT-based models perform most accurately in the image analysis task of image segmentation on training/test datasets comprising real water images. This finding is true for test dataset 1, which comprises only in-distribution images (i.e., all water images share similar patterns to those as the training data, such as all acquired with comparable lighting conditions) and for training dataset 2, which comprises OoD images (i.e., water images include previously unseen characteristics, such as a new lighting condition). It is for this reason that a DPT-based architecture is chosen as a suitable candidate benchmark to assess the efficacy of NCI in image segmentation.

Benchmarking in this example involves preparation of a small and a large real and synthetic mixed dataset. The small training dataset used in the example comprises approximately 4,000 water images (including approximately 2,000 real images and 2,000 synthetic images); the large training dataset used in the example comprises 12,000 water images (including approximately 2,000 real image and 10,000 synthetic images). In line with general consensus that the performance of deep neural networks (such as DPT-based model) is known to scale with training dataset sizes, the DPT model trained with larger benchmark mixed dataset provides greater accuracy when testing with both in-distribution and OoD data. The DPT benchmark trained with a greater number of synthetic images demonstrates a 0.06 and 0.12 increase in accuracy relative to the DPT benchmark trained with fewer synthetic images for both test dataset 1 and test dataset 2, respectively.

The NCI-DPT model (that is, a DPT model incorporating a trained NCI encoder, trained in accordance with techniques disclosed herein) demonstrates improved accuracy relative to both benchmark models in the case of OoD test data. The number of real images in the mixed training dataset, comprising both real and synthetic images, includes half the number of synthetic images (that is, real: synthetic = 1 : 2) and includes approximately 2,000 real images and 4,000 synthetic images. Note the very slight drop in performance in the In-Dist segmentation accuracy relative to the benchmark DPT model; the inventors theorise that this is due to imbalance between real and synthetic images in the dataset.

Oversampling may be used to balance training datasets. That is, datasets comprising real and synthetic data (both small and large) may include a disproportionate number of real images relative to synthetic images. For example, in the event oversampling may be used to duplicate the number of real images, such that the number of real images in the mixed training dataset matches the number of synthetic images. This is advantageous to avoid overfitting of the model to disproportionately synthetic datasets. The NCI-DPT model trained with a greater number of synthetic images, in a balanced manner, demonstrates a 0.05 and 0.60 increase in accuracy relative to the NCI-DPT model trained with fewer synthetic images for both test dataset 1 and test dataset 2, respectively.

Notably, the accuracy of the NCI-DPT model (regardless of training dataset size) exceeds the accuracies of all known models in the accurate segmentation of OoD test images.

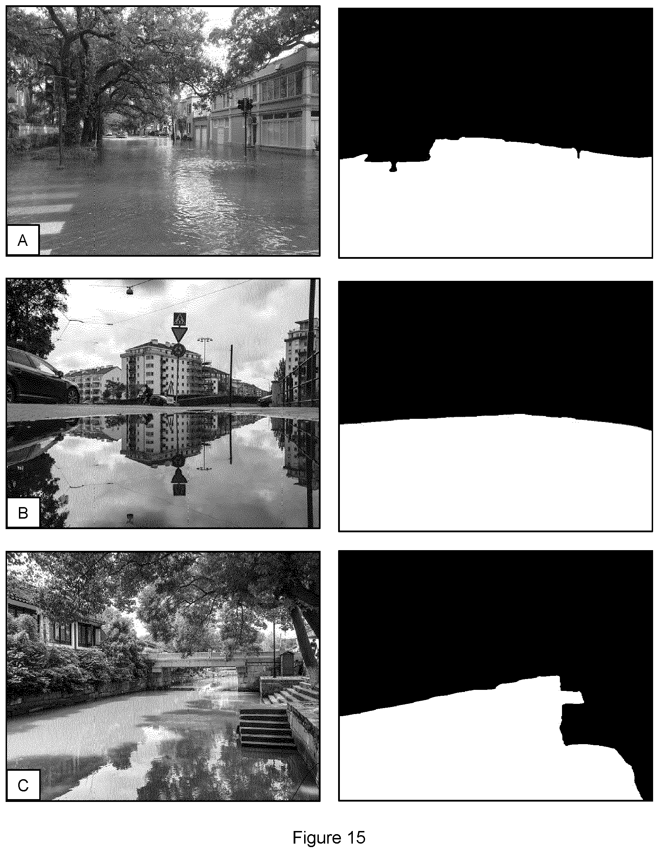

FIGURE 15 provides a qualitative demonstration of NCI when employed in a DPT model, for water segmentation. The left-hand column includes three figures from the ATLANTIS data set (the reader is referred to the work of Erfani, S. et al, ATLANTIS: A benchmark for semantic segmentation of waterbody images. Environmental Modelling & Software, 105333) (note that the original images include colour information). As shown, qualitatively, the DPT model incorporating a trained NCI encoder, trained in accordance with techniques disclosed herein, is well-suited for image segmentation for urban flood images (panel A), for images containing reflective water (panel B), and for images containing murky water (panel C).

In the context of water images, image segmentation data may be processed to provide a comparison to a threshold. In the event that the threshold is exceeded, computational means may output an alarm to indicate the presence of excessive water. The alarm may be a signal, for transmission to the relevant authorities. As a simple example, real-time images acquired from a video surveillance camera may be fed to computational means for processing using an NCI-DPT model; a predefined threshold may be set to indicate that a safe level of water present in the image may correspond to 10% of the field of view. When an image segment, as produced using the NCI-DPT model, occupies greater than 10% of the field of view or image frame, the computational means may be configured to output an audible alarm, or may be configured to transmit a signal to emergency service personnel.

To summarise, techniques herein demonstrate that training with synthetic data is an effective approach for achieving OoD generalisation in such use cases as water segmentation and medical image classification. The NCI approach for efficiently combining synthetic data with real data provides state-of-the-art OoD water segmentation performance. In addition, techniques herein provide a framework for leveraging NCI for improving performance in multi-modal genomics use-cases, where genetic is available only during training, and not during inference.

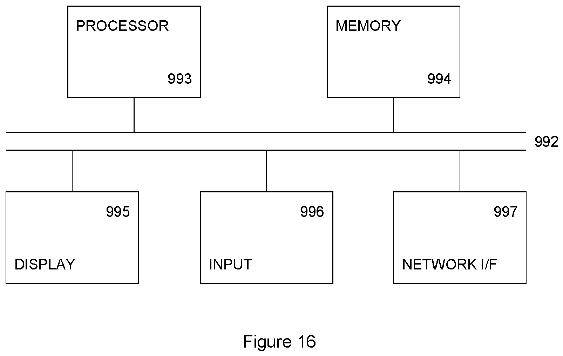

FIGURE 16 is a block diagram of a computing device, such as a data processing server, which embodies the present invention, and which may be used to implement a method of an embodiment of training a machine-learning model for image analysis and/or a method of an embodiment of image analysis. The computing device comprises a processor 993, and memory, 994. Optionally, the computing device also includes a network interface 997 for communication with other computing devices, for example with other computing devices of invention embodiments.

For example, an embodiment may be composed of a network of such computing devices. Optionally, the computing device also includes one or more input mechanisms such as keyboard and mouse 996, and a display unit such as one or more monitors 995. The components are connectable to one another via a bus 992.

The memory 994 may include a computer readable medium, a term which may refer to a single medium or multiple media (e.g., a centralized or distributed database and/or associated caches and servers) configured to carry computer-executable instructions or have data structures stored thereon. Computer-executable instructions may include, for example, instructions and data accessible by and causing a general purpose computer, special purpose computer, or special purpose processing device (e.g., one or more processors) to perform one or more functions or operations. Thus, the term "computer-readable storage medium" may also include any medium that is capable of storing, encoding or carrying a set of instructions for execution by the machine and that cause the machine to perform any one or more of the methods of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing code stored in the memory to implement the various different functions of machine-learning models and components thereof as described here and in the claims. The memory 994 stores data being read and written by the processor 993. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an ASIC, a FPGA, a digital signal processor (DSP), network processor, or the like. In one or more embodiments, a processor is configured to execute instructions for performing the operations and steps discussed herein.

The display unit 995 may display a representation of data stored by the computing device and may also display a cursor and dialog boxes and screens enabling interaction between a user and the programs and data stored on the computing device. The input mechanisms 996 may enable a user to input data and instructions to the computing device.

The network interface (network I/F) 997 may be connected to a network, such as the Internet, and is connectable to other such computing devices via the network. The network I/F 997 may control data input/output from/to other apparatus via the network. Other peripheral devices such as microphone, speakers, printer, power supply unit, fan, case, scanner, trackerball, etc. may be included in the computing device.

An encoder training module may comprise processing instructions stored on a portion of the memory 994, the processor 993 to execute the processing instructions, and a portion of the memory 994 to store encoder weights, biases, and other information concerning the encoder architecture during the execution of the processing instructions. The final trained weights and biases of the encoder may be stored on the memory 994 and/or on a connected storage unit, and may be transmitted, transferred or otherwise communicated to further components.

A discriminator training module may comprise processing instructions stored on a portion of the memory 994, the processor 993 to execute the processing instructions, and a portion of the memory 994 to store discriminator weights, biases, and other information concerning the discriminator architecture during the execution of the processing instructions. The final trained weights and biases of the discriminator may be stored on the memory 994 and/or on a connected storage unit, and may be transmitted, transferred or otherwise communicated to further components.

An image analysis model training module may comprise processing instructions stored on a portion of the memory 994, the processor 993 to execute the processing instructions, and a portion of the memory 994 to store weights, biases, and other information concerning the architecture of the image analysis machine-learning model during the execution of the processing instructions. The final trained weights and biases of the model may be stored on the memory 994 and/or on a connected storage unit, and may be transmitted, transferred or otherwise communicated to further components.

Methods embodying the present invention may be carried out on a computing device such as that illustrated in Figure 16. Such a computing device need not have every component illustrated in Figure 16, and may be composed of a subset of those components. Methods embodying the present invention may be carried out by a single computing device in communication with one or more data storage servers via a network. The computing device may be a data storage itself storing trained model weights and/or biases, training datasets, model hyperparameters, testing images, segmentation and/or classification outputs, etc.

A method embodying the present invention may be carried out by a plurality of computing devices operating in cooperation with one another. One or more of the plurality of computing devices may be a data storage server storing at least a portion of trained model weights and/or biases, training datasets, model hyperparameters, testing images, segmentation and/or classification outputs, etc.

The various methods described above may be implemented by a computer program. The computer program may include computer code (e.g., instructions) arranged to instruct a computer to perform the functions of one or more of the various methods described above. For example, the steps of the methods described in relation to Figure 7 and/or Figure 10 may be performed by the computer code. The steps of the methods described above may be performed in any suitable order. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions may also reside, completely or at least partially, within the memory 913 and/or within the controller circuitry 911 during execution thereof by the computing system 910, the memory 913 and the controller circuitry 911 also constituting computer-readable storage media.

In an implementation, the modules, components and other features described herein may be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components may be implemented as firmware or functional circuitry within hardware devices. Further, the modules and components may be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as "receiving", "determining", "comparing ", "enabling", "maintaining", "identifying", "obtaining", "accessing", or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

While certain embodiments have been described, these embodiments have been presented by way of example only, and are not intended to limit the scope of the inventions. Indeed, the novel methods and apparatuses described herein may be embodied in a variety of other forms; furthermore, various omissions, substitutions and changes in the form of methods and apparatus described herein may be made.

**Claims**

1. A computer-implemented method of training a machine-learning model for image analysis, the method comprising:

   receiving an input training dataset comprising real training data corresponding to a real modality and augmented training data;
   iteratively training an encoder of the machine-learning model to obtain a trained encoder, the iterative training comprising:

   training a discriminator model by minimising a discriminator loss function using the input training dataset to obtain a trained discriminator, the trained discriminator being configured to discriminate whether input data is real data or augmented data; and
   training the encoder by maximising a discriminator error using the training dataset and the trained discriminator to obtain a trained encoder, the trained encoder being configured to invariantly encode real data and augmented data to a real representation space, such that representations preserve information about the real modality.

2. The method of claim 1, wherein the machine-learning model comprises further downstream layers, the further downstream layers with the trained encoder being configured by training to output analysis results of input data.

3. The method of claim 2, further comprising iterative training of the further downstream layers by a multi-objective optimisation procedure using the training dataset and the trained encoder to obtain trained further downstream layers.

4. The method of claim 2 or claim 3, wherein the further downstream layers comprise transformer encoders, reassembly modules, and fusion modules.

5. The method of any preceding claim, further comprising:

   receiving an input image; and
   processing the input image using the trained machine-learning model to obtain analysis results of the input image.

6. A computer implemented method of image analysis, the method comprising:

   receiving an input image;
   processing the input image using a trained machine-learning model to obtain analysis data of the input image, wherein the trained machine-learning model comprises:

      a trained encoder, iteratively trained using an input training dataset comprising real training data corresponding to a real modality and augmented training data, the iterative training having comprised:

         training a discriminator model by minimising a discriminator loss function using the input training dataset to obtain a trained discriminator, the trained discriminator being configured to discriminate whether input data is real data or augmented data; and
         training the encoder by maximising a discriminator error using the training dataset and the trained discriminator to obtain the trained encoder, the trained encoder being configured to invariantly encode real data and augmented data to a real representation space, such that representations preserve information about the real modality; and

      trained further downstream layers, trained by a multi-objective optimization procedure using the training dataset and the trained encoder.

7. The method of any preceding claim, wherein the machine-learning model is for water image segmentation, the input training dataset comprising real training water images and simulated training water images.

8. The method of claim 7, further comprising:

   receiving an input water image in real-time; and
   processing the input water image using the trained machine-learning model to obtain water segmentation results of the input water image.

9. The method of claim 8, further comprising, responsive to determining that the water segmentation results exceed a threshold, outputting an alarm.

10. The method of any of claims 1 to 6, wherein the machine-learning model is for medical image segmentation or classification, the input training dataset comprising training medical images and corresponding genomic training data.

11. The method of claim 10, further comprising:

   receiving an input medical image in real-time; and
   processing the input medical image using the trained machine-learning model to obtain medical segmentation or classification results of the input medical image.

12. The method of claim 11, further comprising, responsive to determining that the medical segmentation or classification results exceed a threshold, outputting an alarm.

13. A data processing apparatus comprising:

   a memory storing computer-executable instructions to carry out the method of any of claims 1 to 12; and
   a processor configured to execute the instructions.

14. A computer program comprising instructions which, when the program is executed by a computer, cause the computer to carry out the method of any of claims 1 to 12.

15. A non-transitory computer-readable storage medium comprising instructions which, when executed by a computer, cause the computer to carry out the method of any of claims 1 to 12.

**Amended claims in accordance with Rule 137(2) EPC.**

1. A computer-implemented method of training a machine-learning model for image analysis, the method comprising:

   receiving an input training dataset comprising labelled real training image data corresponding to a real modality and augmented training data;
   iteratively training an encoder of the machine-learning model to obtain a trained encoder, the iterative training comprising:

   training a discriminator model by minimising a discriminator loss function using the input training dataset to obtain a trained discriminator, the trained discriminator being configured to discriminate whether input data is real image data or augmented data; and
   training the encoder by maximising a discriminator error using the training dataset and the trained discriminator to obtain a trained encoder, the trained encoder being configured to invariantly encode real image data and augmented data to a representation space, such that representations preserve information about the real modality.

2. The method of claim 1, wherein the machine-learning model comprises further downstream layers, the further downstream layers with the trained encoder being configured by training to output analysis results of input data.

3. The method of claim 2, further comprising iterative training of the further downstream layers by a multi-objective optimisation procedure using the training dataset and the trained encoder to obtain trained further downstream layers.

4. The method of claim 2 or claim 3, wherein the further downstream layers comprise transformer encoders, reassembly modules, and fusion modules.

5. The method of any preceding claim, further comprising:

   receiving an input image; and
   processing the input image using the trained machine-learning model to obtain analysis results of the input image.

6. A computer implemented method of image analysis, the method comprising:

   receiving an input image;
   processing the input image using a trained machine-learning model to obtain analysis data of the input image, wherein the trained machine-learning model comprises:

   a trained encoder, iteratively trained using an input training dataset comprising labelled real training image data corresponding to a real modality and augmented training data, the iterative training having comprised:

   training a discriminator model by minimising a discriminator loss function using the input training dataset to obtain a trained discriminator, the trained discriminator being configured to discriminate whether input data is real image data or augmented data; and
   training the encoder by maximising a discriminator error using the training dataset and the trained discriminator to obtain the trained encoder, the trained encoder being configured to invariantly encode real image data and augmented data to a representation space, such that representations preserve information about the real modality; and

   trained further downstream layers, trained by a multi-objective optimization procedure using the training dataset and the trained encoder.

7. The method of any preceding claim, wherein the machine-learning model is for water image segmentation, the input training dataset comprising real training water images and simulated training water images.

8. The method of claim 7, further comprising:

   receiving an input water image in real-time; and
   processing the input water image using the trained machine-learning model to obtain water segmentation results of the input water image.

9. The method of claim 8, further comprising, responsive to determining that the water segmentation results exceed a threshold, outputting an alarm.

10. The method of any of claims 1 to 6, wherein the machine-learning model is for medical image segmentation or classification, the input training dataset comprising training medical images and corresponding genomic training data.

11. The method of claim 10, further comprising:

   receiving an input medical image in real-time; and
   processing the input medical image using the trained machine-learning model to obtain medical segmentation or classification results of the input medical image.

12. The method of claim 11, further comprising, responsive to determining that the medical segmentation or classification results exceed a threshold, outputting an alarm.

13. A data processing apparatus comprising:

   a memory storing computer-executable instructions to carry out the method of any of claims 1 to 12; and
   a processor configured to execute the instructions.

14. A computer program comprising instructions which, when the program is executed by a computer, cause the computer to carry out the method of any of claims 1 to 12.

15. A non-transitory computer-readable storage medium comprising instructions which, when executed by a computer, cause the computer to carry out the method of any of claims 1 to 12.

Figure 1

EP 4 528 541 A1

| Best case scenario | Reflective water | Things in water | Frothy water | Muddy, murky water | Camera with raindrops |
|---|---|---|---|---|---|
| | | | | | |
| Snow | Rain / Fog | Infrared lighting | Night time | ISO Noise | |
| | | | | | |

Figure 2

Clear water    Slightly murky    Murky / Muddy

Figure 3

$T_1$: Transformer
$F_i$: Fusion
$R_i$: Reassemble

Figure 4

Modality Shift

Modality Shift

Figure 5

Figure 6

Receive an input training dataset comprising real training data and augmented training data.　　S71

Iteratively train an encoder of the machine-learning model to obtain a trained encoder.　　S72

S72

Train a discriminator model by minimising a discriminator loss function using the input training dataset to obtain a trained discriminator, the trained discriminator being configured to discriminate whether input data is real data or augmented data　　S721

training the encoder by maximising a discriminator error using the training dataset and the trained discriminator to obtain a trained encoder, the trained encoder being configured to invariantly encode real training data and augmented training data to a same [real] latent spa　　S722

Figure 7

Figure 8

Train NCI

Non-Commutative Invariance (NCI)

Genomic + Imaging Training Data

Sample Train Set Datapoint

Compute Initial Embeddings

NCI upon convergence

Predict Modality

Compute $L_D$ and Update Discriminator

Is non-genomic?

Yes

No

Compute $L_E$ and update Initial Encoder

Propagate Through Later Layers

Prediction

Compute Loss and Update Weights

Until Convergence

Figure 9

Receive an input image.   S101

Process the input image using a trained machine-learning model to obtain analysis data of the input image, wherein the trained machine-learning model comprises:

  a trained encoder, iteratively trained using an input training dataset comprising real training data and augmented training data, the iterative training having comprised:

    training a discriminator model by minimising a discriminator loss function using the input training dataset to obtain a trained discriminator, the trained discriminator being configured to discriminate whether input data is real data or augmented data; and   S102

    training the encoder by maximising a discriminator error using the training dataset and the trained discriminator to obtain the trained encoder, the trained encoder being configured to invariantly encode real data and augmented data to a same latent space; and

  trained further downstream layers, trained by a multi-objective optimization procedure using the training dataset and the trained encoder.

Figure 10

Test NCI

Figure 11

Test NCI

Figure 12

Figure 13a

Figure 13b

Figure 13c

Figure 14

Figure 15

| PROCESSOR | | MEMORY | |
| --- | --- | --- | --- |
| | 993 | | 994 |

992

| DISPLAY | 995 | INPUT | 996 | NETWORK I/F | 997 |
| --- | --- | --- | --- | --- | --- |

Figure 16

Europäisches Patentamt

European Patent Office

Office européen des brevets

# EUROPEAN SEARCH REPORT

**Application Number**

EP 23 19 8408

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JAIN RAHUL KUMAR ET AL: "Unsupervised Logo Detection Using Adversarial Learning From Synthetic to Real Images", IEEE TRANSACTIONS ON EMERGING TOPICS IN COMPUTATIONAL INTELLIGENCE, [Online] vol. 8, no. 1, 31 March 2023 (2023-03-31), pages 710-723, XP093131836, ISSN: 2471-285X, DOI: 10.1109/TETCI.2023.3256475 Retrieved from the Internet: URL:https://ieeexplore.ieee.org/stampPDF/getPDF.jsp?tp=&arnumber=10090408&ref=aHR0cHM6Ly9zY2hvbGFyLmdvb2dsZS5jb20v> [retrieved on 2024-02-15] * the whole document * ----- | 1-15 | INV. G06F16/55 G06N3/045 G06N3/084 G06N3/09 G06N3/094 G06V10/82 |

**TECHNICAL FIELDS SEARCHED (IPC)**

G06F
G06N
G06V

The present search report has been drawn up for all claims

| Place of search | Date of completion of the search | Examiner |
|---|---|---|
| The Hague | 7 March 2024 | Ntarlagiannis, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GANIN, Y. et al.** Domain-adversarial training of neural networks. *J. Mach. Learn. Res.*, January 2016, vol. 17 (1), 2096-2030

- **ERFANI, S. et al.** ATLANTIS: A benchmark for semantic segmentation of waterbody images. *Environmental Modelling & Software*, 105333